# EUROPEAN PATENT APPLICATION

(11) **EP 4 582 034 A1**
(43) Date of publication of application: **09.07.2025**
(21) Application number: 22931714.4
(22) Date of filing: 31.08.2022
(51) Int. Cl.: A61B 17/122, A61B 17/128

(54) **INSERTION-TYPE TISSUE CLIPPING DEVICE**

(71) Applicant: Ningbo Xinwell Medical Technology Co., Ltd., Cixi, Zhejiang 315334 (CN)
(72) Inventor: HUANG, Junjun, Cixi, Zhejiang 315334 (CN); SHAN, Jian, Cixi, Zhejiang 315334 (CN); WU, Hailiang, Cixi, Zhejiang 315334 (CN); CHEN, Qingye, Cixi, Zhejiang 315334 (CN); SUN, Zhongli, Cixi, Zhejiang 315334 (CN)
(74) Representative: Murgitroyd & Company
(86) International application number: PCT/CN2022/116302
(87) International publication number: WO 2023/173693

(57) **Abstract**

An insertion-type tissue clipping device. Clamping heads and bendable portions remain relatively fixed to each other. Each clamping head is opened and closed by means of a bending function of each bendable portion. **In** the device, a moving member is arranged in a clamping member in such a manner that the moving member can move in the axial direction of the clamping member. The device dispenses with a sleeve in the prior art, and the moving member directly drives a clamping arm, such that the clamping arm is opened and closed in combination with a deformation state of the bendable portion. With the same requirement for an opening amplitude, the length of the clamping arm is shorter than the combined length of a clamping arm and the sleeve in the prior art.

## Description

### TECHNICAL FIELD

The present application relates to the field of medical instruments, particularly a structure of an insertion-type tissue-clamping device for surgery.

### BACKGROUND

An insertion-type tissue-clamping device is an insertion-type medical instrument for clamping tissue in a human or animal body to play a role of hemostasis or closure, which includes a hemostatic clamp, a tissue clamp, and the like.

For example, during minimally invasive treatment on gastrointestinal diseases, the tissue-clamping device is generally disposed in the instrument channel of an endoscope to realize therapeutic purposes. For instance, the hemostatic clamp (or the tissue clamp) has been widely used for hemostasis or closure at the site of gastrointestinal bleeding or vulnus.

In the prior art, a type of hemostatic clamp (or tissue clamp) is opened and clamped primarily by the cooperation between the clamp arms and the sleeve. In particular, the left and right clamp arms are loosely assembled together by a pin. When the clamp arm assembly is pulled in a proximal direction, the clamp arms are gradually retracted into the sleeve and engaged with the front edge of the sleeve. Limited by the outer diameter of the sleeve, the sleeve applies a reverse squeezing force to the clamping arms, which enables the clamping arms to be elastically deformed inward, thereby closing. When the clamping arm assembly moves towards a distal direction, the clamping arms are pushed out of the sleeve and automatically reopens due to their elastic restoring force, thereby enabling the clamping device to be repeatedly opened and closed. Due to the utilization of the space in the axial direction of the sleeve, the structure realizes the closure of the clamping arms, and therefore a part of the clamping arms must be contracted in the sleeve, resulting in a long overall length retained in the patient's body after the hemostatic clamp is separated, which is prone to cause damage and discomfort to the patient.

With respect to another type of hemostatic clamp (or the tissue clamp), the clamping arms are connected mainly by a rotating shaft, an up-and-down sliding track is disposed in the sleeve, and the shaft can slide along the track. The upper end of the sleeve is also provided with a fixed shaft, the clamping arm is provided with a long strip-shaped hole, and the fixing shaft extends through the long strip-shaped hole of the clamping arm. By pushing and pulling the sliding shaft, the two clamping arms are driven to move up and down, and forced to move along the path of the long strip-shaped hole after being hindered by the fixed shaft, so as to realize opening and closing. This structure improves operation precision and reduces the size of the clamp, but with more parts, a complicated structure, and a high cost. The overall length of the clamp is still large under the premise of the same opening width. The overall size of the clamp is still long, which is not conducive to passing through the instrument channel of the endoscope, and leads to an obvious foreign body sensation when the whole clamp stays in the human body.

### SUMMARY

### Technical problem

The present application provides an insertion-type tissue clamping device to demonstrate a new opening and clamping structure.

### Solutions to address the problem

### Technical solutions

In view of the above-mentioned object, an embodiment of the present application provides an insertion-type tissue clamping device, including:
a clamping member including at least two clamping arms and a separate base connected to the clamping arms, wherein the clamping arm includes a clamping head and a bendable portion, the clamping head and the bendable portion remain relatively fixed to each other, and the clamping head is configured to clamp a target tissue;
a movement-controlling assembly including a moving member and at least two rocker arms, wherein the moving member is provided in the clamping member in such a way that the moving member is capable of moving in an axial direction of the clamping member, one end of the rocker arm is hinged on the moving member, and another end thereof is connected to one of the clamping arms, respectively;
a transmission assembly including a sleeve assembly and a transmission member, wherein the transmission member is connected to the moving member, and the separate base is connected to the sleeve assembly; and
a control handle connected to the sleeve assembly, wherein the control handle and the transmission member form a linkage structure to control movements of the transmission member and the moving member;
wherein the transmission member has a first stroke, a second stroke, and a third stroke; in the first stroke, the transmission member drives the clamping heads to be away from each other, such that the clamping arms move to be in an opening state; in the second stroke, the transmission member drives the clamping heads to approach each other, such that the clamping arms move to be in a clamping state to clamp the target tissue; and in the third stroke, the clamping arms remain in the clamping state, and the clamping arms are separated from the transmission member and the separate base.

In an embodiment, the clamping member includes a support arm located between the clamping arms, and the support arm is provided with a limiting member; when the moving member moves to a back end, the limiting member is located on a movement path of the rocker arms, and the limiting member is configured to contact at least one rocker arm and form a fulcrum of a lever structure for the at least one rocker arm.

In an embodiment, at least two rocker arms are arranged in an intersection-shape, and the limiting member is located in an intersection region formed by the two rocker arms and adjacent to the moving member, such that when the moving member moves to the back end, the limiting member forms the fulcrum of the lever structure for the at least one rocker arm.

In an embodiment, when the clamping arms move to be in the clamping state, a lever arm of a rotational center of the rocker arm relative to the moving member from a center of the limiting member is longer than a lever arm of a connection center between the rocker arm and the clamping head from the center of the limiting member.

In an embodiment, the two rocker arms have locking fitting surfaces, when the clamping arms are in the clamping state, the locking fitting surfaces of the two rocker arms enclose a locking groove, and the limiting member is located in the locking groove to prevent the two clamping arms from cross moving transversely, so as to maintain the clamping arms in the clamping state.

In an embodiment, the two rocker arms have guide surfaces. When the clamping arms in the opening state move to be in the clamping state, the guide surfaces enclose a guide space. The limiting member is provided in the guide space, and the guide space is in communication with the locking groove to guide the locking groove to be engaged with the limiting member when the rocker arms drive the clamping arms to move to be in the clamping state.

In an embodiment, a front end of the moving member has a limiting structure, when the clamping arms are in the opening state, the limiting structure forms a limitation on a back side of the limiting member to prevent the moving member from continuing to move towards a front end, so as to limit an opening angle of the clamping arms.

In an embodiment, a front end of the rocker arm away from a corresponding clamping arm has a concave portion, and the concave portion is configured to avoid a tissue clamped by the clamping heads.

In an embodiment, one of the moving member and the rocker arm has a rotating shaft, and another one has a self-adjusting groove, the rotating shaft is provided in the self-adjusting groove, and the rotating shaft and the self-adjusting groove are capable of adaptively adjusting relative positions of the moving member and the rocker arm in a transverse direction during movement of the rocker arm.

In an embodiment, at least one support arm has a limiting guide portion provided along a longitudinal direction thereof, the moving member has at least one limiting guide engaging portion, and the limiting guide engaging portion is engaged with the limiting guide portion to prevent the moving member from moving along a direction defined by the limiting guide portion.

In an embodiment, the limiting member has two limiting blocks provided in an axial direction thereof. A limiting space is formed between the two limiting blocks. The two rocker arms are stacked in the limiting space in the axial direction of the limiting member to prevent the two rocker arms from disengaging along the axial direction of the limiting member.

In an embodiment, the bendable portion has at least one deformable portion, when the clamping arms are in the clamping state, the deformable portion is capable of being extruded inwardly and deformed, so as to drive the clamping arms as a whole to move towards a back end of the insertion-type tissue clamping device.

In an embodiment, the clamping member has a locking engaging portion, the moving member has a locking portion; when the clamping arms are separated from the transmission member, the locking portion and the locking engaging portion are locked to prevent the moving member and the rocker arms from moving towards the front end of the clamping member and prevent the clamping arms from opening.

In an embodiment, the locking portion is an engaging platform provided on the moving member, the locking engaging portion is an elastic buckle extending inwardly, and the elastic buckle is provided in a path in which the engaging platform moves towards the back end, when the clamping arms are separated from the transmission member, the elastic buckle abuts against a front side of the engaging platform to prevent the engaging platform from moving towards the front end of the clamping member.

In an embodiment, a front end of the transmission member has a first snap portion, and a back end of the moving member has a second snap portion; the first snap portion is snap-fitted with the second snap portion, at least one of the first snap portion and the second snap portion is of a deformable structure; when the transmission member moves along the third stroke, the deformable structure is capable of deforming under the pulling force of the transmission member, such that the first snap portion is disengaged from the second snap portion, and the moving member is separated from the transmission member.

### Beneficial effects of the present application

### Beneficial effects

The insertion-type tissue clamping device according to the above embodiments, the clamping head and the bendable portion remain relatively fixed to each other, and the opening and closing of the clamping heads is achieved by the bending function of the bendable portion. In this device, the moving member is located in the clamping member in such a way that the moving member can move in the axial direction of the clamping member. In this structure, the sleeve in the existing structure can be omitted, the clamping arms can be directly driven by the moving member, which combines the deformation state of the bendable portion to realize the opening and closing of the clamping arms. Under the same opening width, the length of the clamping arms is shorter than that of the combination of the clamping arms and the sleeve in the prior art.

Further, in an embodiment, the limiting member is further included. When the moving member moves to the back end, the limiting member is located on the movement path of the rocker arms to contact at least one rocker arm and form a fulcrum of a lever structure for the rocker arm. Through the function of the lever structure, the clamping arms can clamp the target tissue in the clamping state more firmly.

### BRIEF DESCRIPTION OF THE DRAWINGS

### Description of the drawings

FIG. 1 is a schematic structural view of an insertion-type tissue clamping device according to an embodiment of the present application, wherein a transmission assembly is simplified.
FIG. 2 is a schematic structural view of clamping arms in an opening state according to an embodiment of the present application.
FIG. 3 and FIG. 4 are cross-sectional views of the clamping member in an opening state according to one embodiment of the present application, wherein the transmission member is in a first stroke, and the movement direction of the transmission member is shown by an arrow.
FIG. 5 is a schematic structural view of clamping arms in a clamping state according to an embodiment of the present application.
FIG. 6 and FIG. 7 are cross-sectional views of a clamping member in a clamping state according to an embodiment of the present application, wherein the transmission member is in a second stroke, and the movement direction of the transmission member is shown by an arrow.
FIG. 8 is an exploded schematic view of each part at a front end according to an embodiment of the present application.
FIG. 9 is a schematic view of a matching structure of rocker arms and a limiting member according to an embodiment of the present application.
FIG. 10 is a schematic view of a matching structure of a limiting member and support arms according to an embodiment of the present application.
FIG. 11 is an exploded schematic view of a mating structure of rocker arms and a moving member according to an embodiment of the present application.
FIG. 12 is a schematic structural view of a rocker arm according to an embodiment of the present application.
FIG. 13 is a schematic structural view of clamping arms in a clamping and self-locking state according to an embodiment of the present application, wherein the transmission member is in a third stroke, and the movement direction of the transmission member is shown by an arrow.
FIG. 14 and FIG. 15 are cross-sectional views of clamping arms in a clamping and self-locking state according to an embodiment of the present application.
FIG. 16 is a schematic structural view of clamping arms in a clamping and locking state according to an embodiment of the present application.
FIG. 17 and FIG. 18 are cross-sectional views of clamping arms is in a clamping and locking state according to an embodiment of the present application, wherein the transmission member is in a third stroke, and the movement direction of the transmission member is shown by an arrow.
FIG. 19 is a schematic view of a cylindrical clamping member according to an embodiment of the present application after being expanded along a longitudinal direction.
FIG. 20 is a schematic structural view of an annular deformation portion according to an embodiment of the present application in a natural state.
FIG. 21 is a schematic structural view of an annular deformation portion according to an embodiment of the present application after being extruded inwardly and deformed.
FIG. 22 is a schematic view showing a deformation direction of a deformation portion when a clamping member clamps a thick tissue according to an embodiment of the present application, wherein the deformation direction is shown by an arrow.
FIG. 23 is a schematic view showing a distance between a limiting member and a locking groove when a clamping member clamps a thick tissue according to another embodiment of the present application.
FIG. 24 is a schematic structural view of two deformation portions according to an embodiment of the present application in a natural state.
FIG. 25 is a schematic structural view of two deformation portions according to an embodiment of the present application after being extruded inwardly and deformed.
FIG. 26 is a schematic structural view of a plurality of deformation portions according to an embodiment of the present application.
FIG. 27 is a schematic structural view showing a moving member is in an inner disengagement from a transmission member, when clamping arms are in a clamping state according to an embodiment of the present application.
FIG. 28 is a cross-sectional view of a moving member and a transmission member after an inner disengagement when clamping arms are in a clamping state, according to an embodiment of the present application, wherein the transmission member is in a third stroke, and the movement direction of the transmission member is shown by an arrow.
FIG. 29 is a schematic cross-sectional view showing a process of an inner disengagement between a moving member and a transmission member according to an embodiment of the present application.
FIG. 30 and FIG. 31 are schematic structural views of a separate base, clamping arms and support arms after an outer disengagement when clamping arms are in a clamping state, according to an embodiment of the present application.
FIG. 32 is a cross-sectional view of a separate base, clamping arms and support arms after an outer disengagement when clamping arms are in a clamping state, according to an embodiment of the present application, wherein the transmission member is in a third stroke, and the movement direction of the transmission member is shown by an arrow.

### DETAILED DESCRIPTION

### Embodiments of the present application

The present application will be described in further detail below with reference to embodiments in conjunction with the accompanying drawings. Similar elements in different embodiments have associated similar element reference numbers. In the following embodiments, many details are described in order to enable a better understanding of the present application. However, those skilled in the art can easily recognize that some of these features can be omitted or replaced with other elements, materials, or methods, in different circumstances. In some cases, some operations related to the present application are not shown or described in the specification, in order to avoid the core part of the present application being neglected by excessive description. It is not necessary for those skilled in the art to describe these related operations in detail, and they can fully understand the related operations based on the description in the specification and general technical knowledge in the art.

In addition, the features, operations, or characteristics described in the specification can be combined in any suitable manner to form various embodiments. Meanwhile, the steps or actions in the description of the method can also be sequentially reversed or adjusted in a manner apparent to those skilled in the art. Accordingly, the various sequences in the specification and drawings are merely for the purpose of clearly describing a particular embodiment, and are not meant to be any particular sequence unless otherwise indicated that a certain order must be followed.

The serial numbers assigned herein to elements, such as "first", "second", etc., are merely used to distinguish the objects described, without any sequential or technical meaning. However, "connection" and "junction" in this application include direct and indirect connection (junction) unless otherwise specified.

The present embodiment provides an insertion-type tissue-clamping device (hereinafter referred to as a clamping device for convenience of description). The clamping device is configured to clamp the tissue (collectively referred to as target tissue) in a human or animal body to play a role of hemostasis or closure, which can include but is not limited to a hemostatic clamp, a tissue clamp, and the like. The clamping device can be a disposable device or a reusable device.

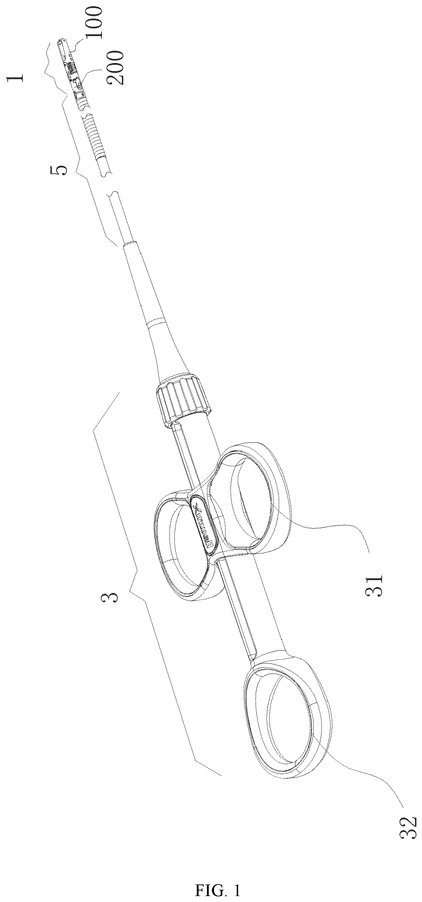

Referring to FIGs. 1 to 7, in an embodiment, the clamping device includes a clamping member 1, a control handle 3, a movement-controlling assembly 4, a transmission assembly 5, and other related components.

The control handle 3 is an operating component for controlling the clamping device. The operator can open and close the clamping member 1 to clamp a target tissue 2 by manually operating the control handle 3. For convenience of description, in the present application, an end where the clamping member 1 is located as a front end in the entire clamping device, and the other end where the control handle 3 is located is defined as a back end, and the front and back directions in other components are all based on this direction.

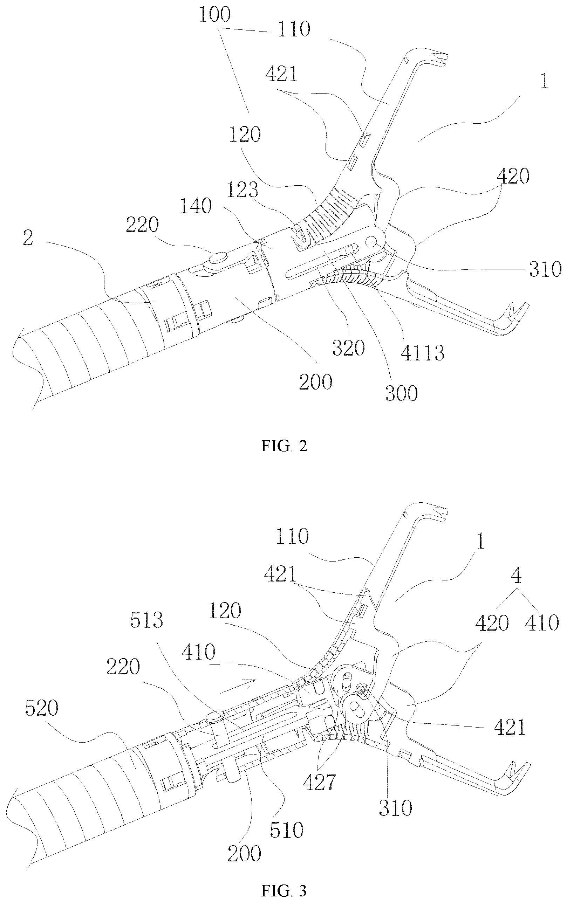

The clamping member 1 includes at least two clamping arms 100 and a separate base 200 connected to the clamping arms 100. This connection can be an integrally formed structure (as shown in the embodiment of FIGs. 2 to 7), and the integrally formed structure can be a target object integrally processed from the same material, rather than being assembled by two or more parts. This integrally formed structure, including other integrally formed structures described below, can be made by, but is not limited to, injection molding, laser cutting, and other machining processes. In particular, when laser cutting is used, processing of extremely small gaps can be realized, which is beneficial to miniaturization of the overall structure and improvement of structural compactness. Further, a clamping head and a bendable portion can be connected as one piece between the clamping arms 100 and the separation base 200 by engagement, welding, adhesion, screw locking, riveting, or other fixing methods.

The clamping arm 100 includes a clamping head 110 configured to clamp the target tissue 2 and a bendable portion 120. The clamping head 110 and the bendable portion 120 remain relatively fixed to each other. This relative fixation can be achieved either by an integrally formed structure (as shown in the embodiment of FIGs. 2 to 7), or can be connected as one piece by engagement, welding, adhesion, screw locking, riveting, or other fixing methods.

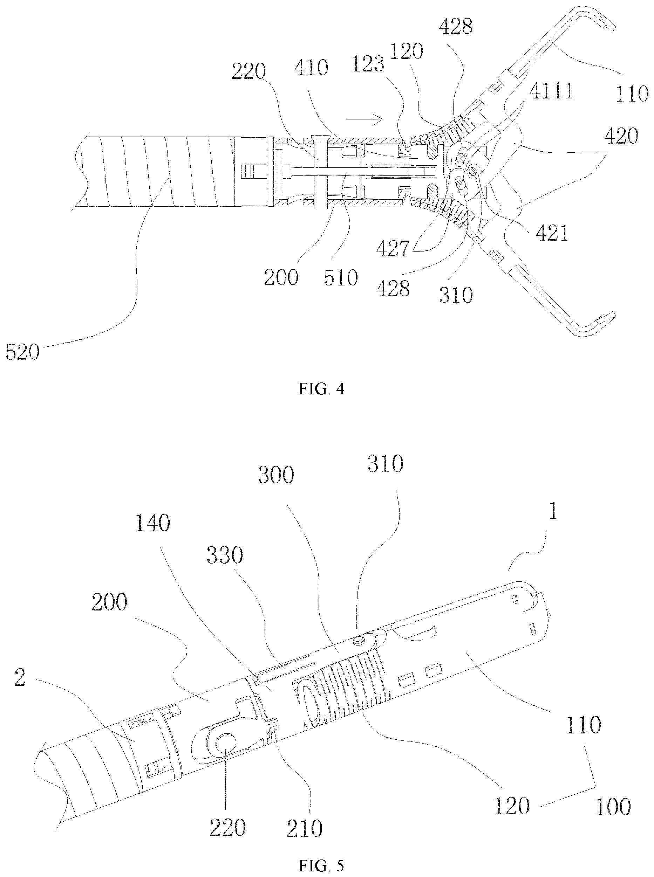

In order to better clamp the target tissue 2, in some embodiments, the clamping arms 100 are provided in a claw-type structure to clamp the target object. The claw-type structure is a structure which can clamp the target object firmly. For example, in the embodiment shown in FIGs. 2 to 7, when two clamping arms 100 are provided, the two clamping arms 100 are arranged opposite to each other. When the clamping arms 100 are closed as shown in FIGs. 5 to 7 (at this time, the clamping arms 100 are in a clamping state), the target object can be clamped. In some other embodiments, when the number of clamping arms 100 is different, they can have different claw-type structures. For example, when the number of clamping arms 100 is three, the three clamping arms 100 can be arranged in a triangle to clamp the target object.

The movement-controlling assembly 4 serves to drive the clamping arms 100 to move in the opening direction and the closing direction. Referring to FIGs. 2 to 7 and 11 to 12, the movement-controlling assembly 4 includes a moving member 410 and at least two rocker arms 420. The moving member 410 is provided in the clamping member 1 in such a manner as to be movable along the axial direction of the clamping member 1. One end of each of the two rocker arms 420 is hinged to the moving member 410, and the other end of each of the two rocker arms 420 is respectively connected to one of the clamping arms 100, for example, by laser or other forms of fixing methods such as engagement, welding, adhesion, screw locking, riveting, etc. For example, referring to FIGs. 2 to 8, in an embodiment, the front end of the rocker arm 420 has a protrusion connecting portion 421, and the protrusion connecting portion 421 is inserted into a corresponding clamping arm 100 and is fixedly connected to the clamping arm 100, for example by ultrasonic welding.

When the moving member 410 moves towards the front end of the clamping device, the two clamping arms 100 can be spread apart by the rocker arms 420, so that the clamping arms 100 are in an opening state. When the moving member 410 moves towards the back end of the clamping device, the two clamping arms 100 can be pulled to approach each other by the rocker arms 420, thereby enabling the clamping arms 100 to move to be closed, and to be in a clamping state.

The transmission assembly 5 includes a transmission member 510 and a sleeve assembly 520. The transmission member 510 is connected to the moving member 410, and the separate base 200 is connected to the sleeve assembly 520, so that the entire clamping member 1 is supported on the sleeve assembly 520. The sleeve assembly 520 is connected to the control handle 3, and the control handle 3 forms a linkage structure with the transmission member 510 to control the movements of the transmission member 510 and the moving member 410.

Referring to FIG. 1, in an embodiment, the control handle 3 can include a control portion 31 configured to control the transmission member 510 and a grasping portion 32 for the operator to grasp. The control portion 31 can move relative to the grasping portion 32. In FIG. 1, the grasping portion 32 has a structure into which an operator's thumb can be inserted, and the control portion 31 can move back and forth with respect to the grasping portion 32. The control portion 31 can be connected to the transmission member 510 through a pulling member to form the linkage structure. The pulling member can be, but is not limited to, steel wire rope, a pulling rope made of other materials, and other components that can be used as the pulling structure of the clamping device. With the pulling member, the operator can drive the transmission member 510 and the moving member 410 to move by the control portion 31, and thus control the opening and closing of the clamping arms 100.

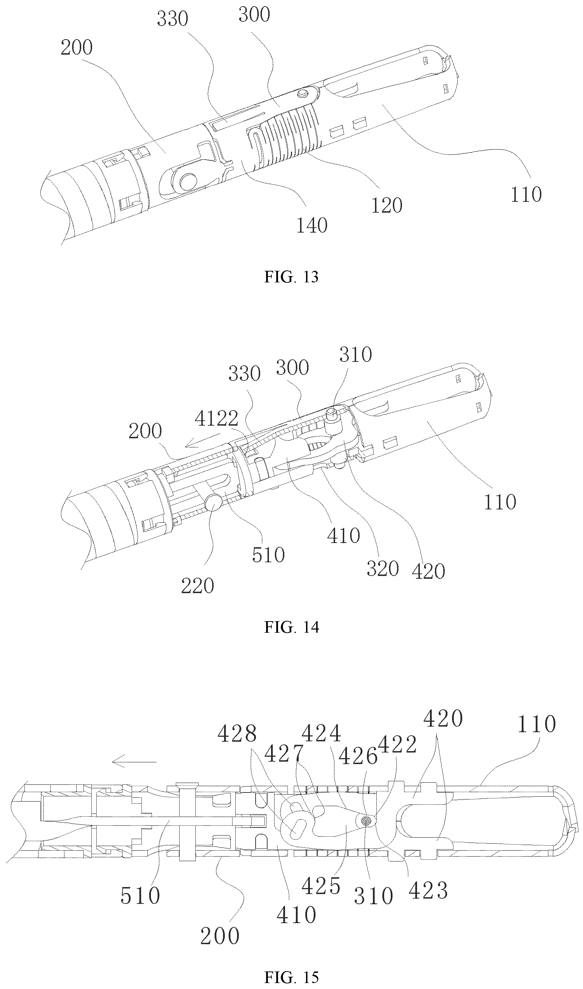

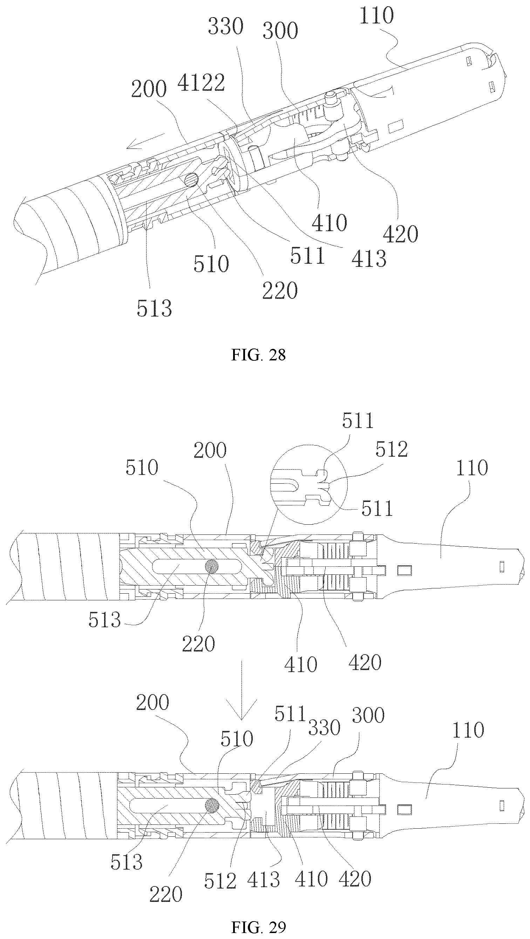

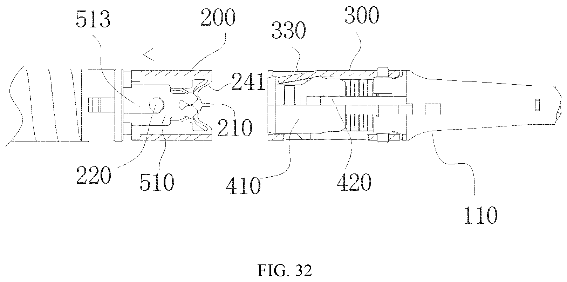

Different movement strokes of the transmission member 510 correspond to different states of the clamping device. Specifically, the transmission member 510 has a first stroke, a second stroke, and a third stroke. In the first stroke, the transmission member 510 drives the clamping heads 110 to be away from each other, such that the clamping arms 100 move to the opening state, as shown in FIGs. 2 to 4. In the second stroke, the transmission member 510 drives the clamping heads 110 to approach each other, such that the clamping arms 100 move to the clamping state to clamp the target tissue 2, as shown in FIGs. 5 to 7. In the third stroke, the clamping arms 100 are locked in the clamping state, and the clamping arms 100 are separated from the transmission member 510 and the separate base 200, as shown in FIGs. 13 to 18 and FIGs. 27 to 32. When the clamping arms 100 are completely separated from the transmission member 510 and the separate base 200, the clamping arms 100 can remain in the patient, and the portion detached from the clamping arms 100 is removed from the patient. The above process is a general introduction to the whole use process of the clamping device.

Referring to FIGs. 2 to 7, in an embodiment, the two rocker arms 420 are arranged in an intersection-shape. Certainly, in some other embodiments, the two rocker arms 420 can also be arranged in other shapes, for example, in a shape of the inverted Chinese character "eight" or a shape of the Arabic number "11" between the moving member 410 and the clamping arms 100.

When the clamping arms 100 clamp the target tissue 2, the target tissue 2 exerts a counter force on the clamping arms 100. Therefore, in order to firmly clamp the target tissue 2, it is necessary to ensure that the clamping device can provide a sufficient occluding force to the clamping arms 100. In view of this problem, referring to FIGs. 2 to 10, in some embodiments, the clamping member 1 further includes a pair of support arms 300 positioned between the two clamping arms 100, and the support arms 300 are connected to the clamping arms 100 and the separate base 200. This connection can be achieved by an integrally formed structure (as shown in the embodiment of FIGs. 2 to 7), or can be connected as one piece by engagement, welding, adhesion, screw locking, riveting, or other fixing methods. The pair of support arms 300 is provided with a limiting member 310, which, as shown in FIG. 4, is located in an intersection region 421 formed by the two rocker arms 420 and adjacent to the moving member 410. Referring to FIGs. 2 to 7, when the moving member 410 moves to the back end, the limiting member 310 is capable of contacting at least one rocker arm 420 at least when the clamping arms 100 are in the clamping state, and forming a fulcrum of the lever structure for the at least one rocker arm 420.

In the embodiment shown in FIGs. 2 to 8, the rocker arm 420 serves not only as a key part connecting the clamping arms 100 to the moving member 410, but also as a part cooperating with the limiting member 310 to achieve the lever structure. To fulfill the above two functions, the outer edge of the rocker arm 420 facing the limiting member 310 needs to be able to generate a force with the limiting member 310. Because the front end of the rocker arm 420 is fixedly connected (such as welded) to the middle of the clamping arm 100, the clamping arm 100 and the rocker arm 420 can be regarded as the same force system at this time, and the limiting member 310 can be used as the lever rotation fulcrum of the clamping arm 100. During the movement of the moving member 410 towards the back end, the displacement of the moving member 410 towards the back end is converted into the closure of the clamping arm 100. At this time, there are both relative sliding displacement and relative rotational displacement between the outer edge of the rocker arm 420 adjacent to the limiting member 310 and the limiting member 310. The pulling force from the control handle 3 is converted into a downward force of the rocker arm 420 relative to the limiting member 310. The acting force can be effectively transmitted to the other end of the rocker arm 420 by the action of the limiting member 310 serving as a lever fulcrum, and then transmitted to the front end of the clamping arm 100, thereby obtaining a relatively great clamping force (occluding force).

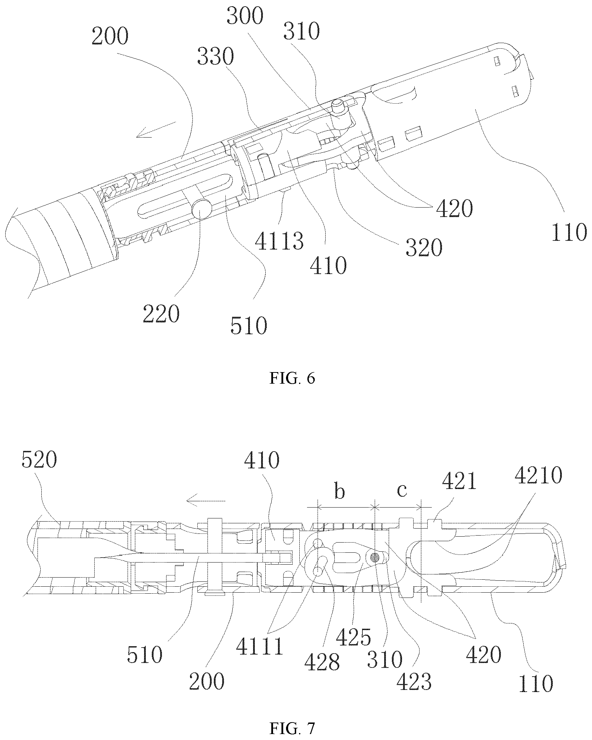

In order to ensure that the lever structure formed by the limiting member 310 and the rocker arm 420 is a labor-saving lever at least when the clamping arms 100 are in the clamping state, therefore, in an embodiment, referring to FIG. 7, at least when the clamping arms 100 are in the clamping state, the lever arm b of the rotational center of the rocker arm 420 relative to the moving member 410 from the center of the limiting member 310 is longer than the lever arm c of the connection center between the rocker arm 420 and the clamping head 110 from the center of the limiting member 310. In this way, when the clamping arms 100 are in the clamping state, the operator can use a more labor-saving manner to enable the clamping arms 100 to obtain a relatively great occluding force.

Certainly, in some other embodiments, at least when the clamping arms 100 are in the clamping state, the lever arm b of the rotational center of the rocker arm 420 relative to the moving member 410 from the center of the limiting member 310 is equal to or less than the lever arm c of the connection center between the rocker arm 420 and the clamping head 110 from the center of the limiting member 310. At this time, the direction of the force is changed mainly by a lever, so that the operator can more easily make the clamping arms 100 better occlude the target tissue 2 by pulling the transmission member 510.

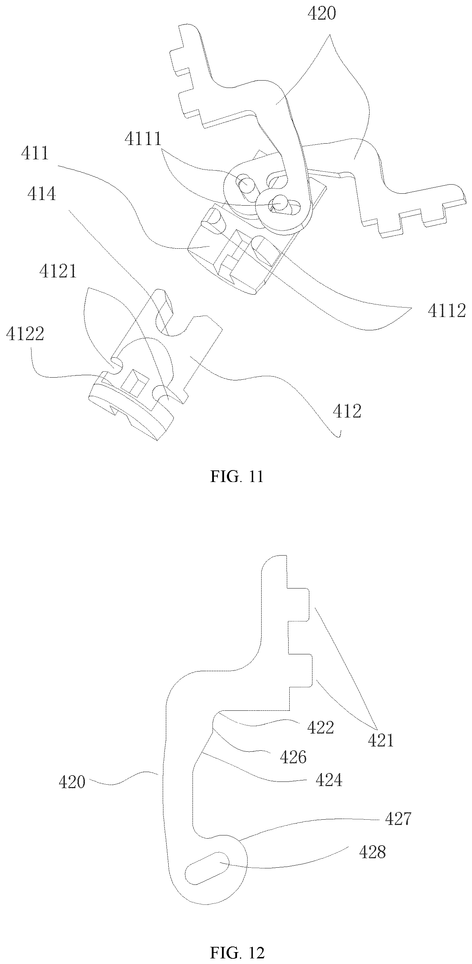

Further, when the clamping arms 100 occlude the target tissue 2, in order to prevent the clamping arm 100 from being spread apart by the counter force from the target tissue 2, in an embodiment, a self-locking structure can be formed by combining the limiting member 310 and the rocker arm 420. Referring to FIGs. 12 and 15, in an embodiment, the two rocker arms 420 have locking fitting surfaces 422. When the clamping arms 100 are in the clamping state, the locking fitting surfaces 422 of the two rocker arms 420 enclose a locking groove 423, and the limiting member 310 is located in the locking groove 423 to prevent the transverse cross movement of the two rocker arms 100, thereby maintaining the clamping arms 100 in the clamping state.

The self-locking principle involves that when the clamping arms 100 are in the clamping state, the counter force of the target tissue 2 spreading apart the clamping arms 100 is converted into the transverse displacement of the rocker arm 420 relative to the limiting member 310 by the rocker arm 420, and the action force on the moving member 410 towards the front end becomes very small. At this time, the limiting member 310 is located in the locking groove 423, which prevents the transverse cross movement of the rocker arms 420, thereby preventing the tissue clamping arms 100 from opening.

In an embodiment, the transverse size of the locking groove 423 is slightly greater than or equal to the transverse size of the limiting member 310, which not only ensures that the limiting member 310 can enter the locking groove 423, but also avoids large transverse displacements of the clamping arms 100 due to too much gap between the locking groove 423 and the limiting member 310, thus preventing the clamping arms 100 from releasing the target tissue 2.

The limiting member 310 remains substantially stationary during the movement of the rocker arms 420 towards the back end. Therefore, in order to guide the locking groove 423 to be engaged with the limiting member 310, in some embodiments, a guide structure can also be provided. Referring to FIGs. 7, 12, and 15, in an embodiment, the two rocker arms 420 have guide surfaces 424. When the clamping arms 100 in the opening state move to the clamping state, the guide surfaces 424 enclose a guide space 425 as the movement of the rocker arms 420. The limiting member 310 is provided in the guide space 425, and the guide space 425 is in communication with the locking groove 423. When the rocker arms 420 moves to the back end and drives the clamping arms 100 to move to be in the clamping state, the guide space 425 guides the locking groove 423 to be engaged with the limiting member 310.

As shown in FIGs. 7 and 15, in an embodiment, the locking groove 423 abuts against the front side of the guide space 425, and the transverse size of the guide space 425 gradually decreases from back to front.

In order to form the guide structures as shown in FIGs. 7 and 15, in an embodiment, referring to FIGs. 7, 12 and 15, the rocker arms 420 have inclined guide surfaces 424. The two inclined guide surfaces 424 can cooperate to form the guide space 425 in a shape like a Chinese character "eight" when the clamping arms 100 in the opening state move to be in the clamping state. When the contact point between the rocker arms 420 and the limiting member 310 passes the turning point of the guide surfaces 424 of the rocker arms 420, the limiting member 310 enters the locking groove 423, and then the clamping arms 100 interlock with the limiting member 310.

Certainly, in some other embodiments, the guide surface 424 can also be of other shapes, thereby forming the guide spaces 425 of other shapes.

To form the self-locking structure as shown in FIGs. 7 and 15, in an embodiment, referring to FIGs. 7, 12 and 15, the rocker arm 420 has an arc-shaped locking fitting surface 422. The locking fitting surface 422 is located on the front side of the guide surface 424. When the clamping arms 100 are in the clamping state, the locking fitting surfaces 422 of the two rocker arms 420 enclose an arc-shaped locking groove 423. When the limiting member 310 has a cylindrical structure (such as a limiting shaft), the arc-shaped locking groove 423 can be more fitted to the limiting member 310.

Referring to FIGs. 12 and 15, in an embodiment, the locking fitting surface 422 further has a liner section 426, and when the clamping arms 100 are in the clamping state, the liner sections 426 of the two locking fitting surfaces 422 can cooperate to form a vertical inlet-outlet opening for the locking groove 423. An extending direction of the vertical inlet-outlet opening is consistent with an axial direction of the moving member 410. The vertical inlet-outlet opening can improve the difficulty of the limiting member 310 sliding out of the locking groove 423.

Certainly, in some other embodiments, the vertical inlet-outlet opening can also be replaced with an inlet-outlet opening in the shape of the Chinese character "eight" with large inside and small outside or in other shapes, so that the limiting member 310 entering the locking groove 423 is more difficult to slide out of the inlet-outlet opening in the shape of the Chinese character "eight".

In an embodiment, the rocker arm 420 can be integrally formed, for example by laser cutting or stamping from metal sheet. In an embodiment, when the metal sheet material is used, the thickness of the rocker arm 420 can be in a range from 0.3 mm and 0.6 mm, so as to ensure the strength of the rocker arm 420 and prevent the rocker arm 420 from being overweight. Alternatively, the rocker arm 420 can also be composed of multiple components that are manufactured separately and then fixedly connected together.

Further, considering that the rocker arms 420 and the moving member 410 need to be able to adjust automatically the relative positions therebetween in some circumstances, for example, during the opening and closing of the clamping arms 100, due to the limitation of the internal space of the clamping member 1, the rocker arms 420 need to realize the complex movement trajectories in a space with a small inner diameter, therefore, the rocker arms 420 are bound to interfere with the clamping member 1 during their movements. In view of this problem, in an embodiment, one of the moving member 410 and the rocker arm 420 has a rotating shaft and the other has a self-adjusting groove, and the rotating shaft is provided in the self-adjusting groove. The rotating shaft and the self-adjusting groove can adaptively adjust the relative positions of the moving member 410 and the rocker arm 420 in the transverse direction during the movement of the rocker arm 420. The inner cavity of the self-adjusting groove is larger than the rotating shaft, so that the rotating shaft can adaptively adjust its position in the self-adjusting groove.

Referring to FIGs. 4 and 7, in an embodiment, the self-adjusting groove 428 is provided on the rocker arm 420, and the rotating shaft 4111 is fixedly provided on the moving member 410. In the illustrated embodiment, the self-adjusting groove 428 is in a strip shape, and the rotating shaft 4111 can move correspondingly in the strip-shaped structure. When the clamping arms 100 are in the clamping state, as shown in FIG. 7, the two self-adjusting grooves 428 are disposed opposite to each other, and the back ends of the self-adjusting grooves 428 are both inclined between the two rocker arms 420, so that the rocker arms 420 can transversely adjust their positions during movement.

Certainly, in some other embodiments, the self-adjusting groove 428 can also be provided on the moving member 410 and the rotating shaft 4111 is provided on the rocker arm 420. In some other embodiments, the self-adjusting groove 428 can also be in other shapes, such as in a circular or elliptical shape having an inner diameter greater than the outer diameter of the shaft 4111.

Referring to FIGs. 4 and 7, in an embodiment, the two rocker arms 420 and the moving member 410 are respectively connected by respective rotating shafts 4111.Rotational axes of the two rocker arms 420 with respect to the moving member 410 are separated and parallel. In some other embodiments, the two rocker arms 420 can also be connected to the moving member 410 through a common rotating shaft 4111.

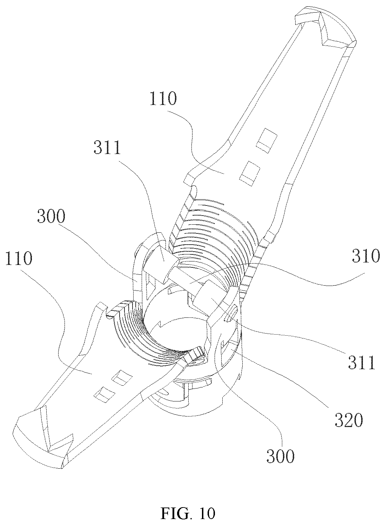

Further, referring to FIG. 10, in an embodiment, a pair of support arms 300 are oppositely disposed at a notch between two clamping arms 100. Both ends of the limiting member 310 are fixedly connected to one of the support arms 300, respectively, for example, by laser or other forms of fixing methods such as engagement, welding, adhesion, screw locking, or riveting to form a stable limiting structure.

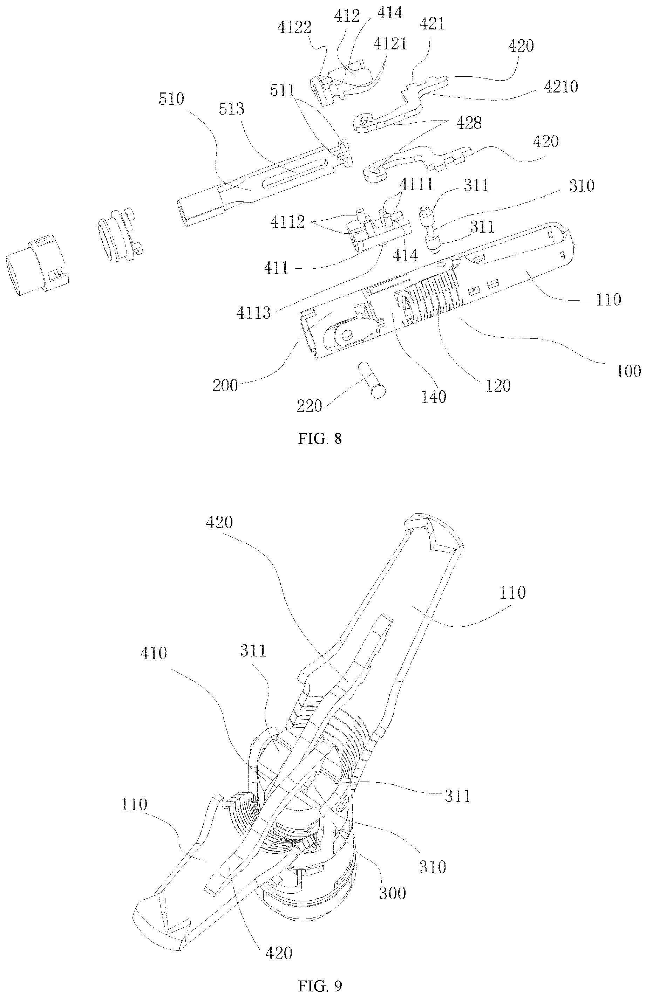

Referring to FIGs. 8 to 10, in an embodiment, the limiting member 310 has two limiting blocks 311 provided along the axial direction thereof. A limiting space is formed between the two limiting blocks 311. The two rocker arms 420 are stacked in the limiting space along the axial direction of the limiting member 310 to prevent the two rocker arms 420 from disengaging along the axial direction of the limiting member 310, and to restrict the rocker arms 420 from sliding outwards beyond both ends, thereby preventing the teeth of the clamping arm 100 from not being accurately aligned.

In the embodiment shown in FIG. 10, the limiting member 310 has a shaft-shaped structure, and the two limiting blocks 311 are distributed in a dumbbell shape on the limiting member 310. Certainly, the limiting member 310 can have other structures in some other embodiments.

Further, the moving member 410 is configured to be able to move in the clamping member 1 and drive the rocker arms 420 to move. The movement of the moving member 410 relative to the clamping member 1 can be, but is not limited to, sliding, rolling, or the like. The moving member 410 may have any shape and structure that meet the above requirements.

Referring to FIGs. 8, 9 and 11, in an embodiment, the moving member 410 is a sliding block, and the moving member 410 is slidably provided in the clamping member 1. The moving member 410 includes a first seat body 411 and a second seat body 412. The rocker arm 420 is connected to and limited between the first seat body 411 and the second seat body 412. The first seat body 411 and the second seat body 412 are spliced to form the moving member 410. In this structure, the moving member 410 includes the first seat body 411 and the second seat body 412 that are manufactured separately to reduce the difficulty of manufacturing. In this assembly structure, the rocker arms 420 are mounted onto the first seat body 411 firstly, and then the second seat body 412 are engaged with the first seat body 411 to complete the assembly of the moving member 410 as well as the mounting of the rocker arms 420.

Referring to FIG. 11, in an embodiment, the first seat body 411 has a rotating shaft 4111, and the rocker arm 420 is sleeved on the rotating shaft 4111. The second seat body 412 can be engaged with the first seat body 411. In order to be engaged with the second seat body 412, the first seat body 411 is provided with a protrusion 4112, and the second seat body 412 is provided with a recessed portion 4121, and the protrusion 4112 is inserted into the recess 4121 to form a transverse positioning.

Certainly, in some other embodiments, the moving member 410 can be of other structures, such as an integrally formed structure or assembled from three or more subcomponents. The rocker arm 420 can be otherwise mounted to the moving member 410.

In some embodiments, the moving member 410 can be formed by laser cutting of steel pipe or by powder metallurgy molding (with a relatively simple assembly).

Further, when the moving member 410 moves towards the front end of the clamping device, the rocker arms 420 can be driven to open the support arms 300, thereby causing the support arms 300 to be switched to the opening state. In order to limit the opening angle of the support arms 300, an angle limiting structure can be provided on the support arms 300, or the angle limiting structure can be provided on the moving member 410 and/or the rocker arms 420.

For example, referring to FIGs. 8 and 11, in an embodiment, the front end of the moving member 410 has the angle limiting structure 414. When the clamping arms 100 are in the opening state, the angle limiting structure 414 forms a limitation on the back side of the limiting member 310 to prevent the moving member 410 from continuing to move towards the front end and limit the opening angle of the clamping arms 100.

In an embodiment, the angle limiting structure 414 is a limiting groove provided on a side of the front end of the moving member 410 away from the corresponding clamping arm 100, for example, both the first seat body 411 and the second seat body 412 have the limiting groove. The limiting groove serves as the angle limiting structure 414. When the clamping arms 100 are in the opening state, the bottom of the limiting groove can be in contact with the limiting member 310, thereby forming a limitation to prevent the moving member 410 from continuing to move towards the front end and limit the opening angle of the clamping arms 100.

In another embodiment, an angle limiting structure can also be formed by rocker arms 420. Referring to FIGs. 2 to 4 and 12, in an embodiment, two rocker arms 420 are connected to one end of the moving member 410 and have a protruded limiting portion 427. When the clamping arms 100 are in the opening state, the two limiting portions 427 form an angle limiting structure, for example, in a cross-shaped or just left-right alignment. The angle limiting structure forms a limitation on the back side of the limiting member 310 to prevent the moving member 410 from continuing to move towards the front end, thereby limiting the opening angle of the clamping arms 100. When the limiting portion 427 is provided, an angle limiting structure may not be provided additionally. The limiting portion 427 is directly provided on the rocker arm 420, which can be integrally formed and manufactured, and has a simple structure, simplifying the structure of the entire clamping device.

Further, referring to FIGs. 7 and 8, in order to avoid the influence of the rocker arms 420 when the target tissue 2 is clamped. In an embodiment, the front end of the rocker arm 420 away from the corresponding clamping arm 100 has a concave portion 4210, and the concave portion 4210 is configured to avoid the tissue clamped by the clamping heads 110.

Further, in an embodiment, in order to prevent the moving member 410 from rotating in the clamping member 1, at least one support arm 300 has a limiting guide portion provided along a longitudinal direction thereof, the moving member 410 has at least one limiting guide engaging portion, and the limiting guide engaging portion cooperates with the limiting guide portion to prevent the movement of the moving member 410 in the direction defined by the limiting guide portion.

In an embodiment, one of the limiting guide portion and the limiting guide engaging portion is a guide groove, and the other is a guide block provided convexly. The guide block extends into the guide groove. For example, referring to FIGs. 6 and 8, in an embodiment, the guide block 4113 is provided on the moving member 410, specifically on the outer wall of the first seat body 411. The guide groove 320 is provided on the support arm 300 corresponding to the first seat body 411. Certainly, when both sides need to be guided, the guide block 4113 can also be provided on the second seat body 412, and the guide groove 320 can be provided on the other support arm 300 corresponding to the second seat body 412.

Further, when the transmission member 510 moves along the second stroke, in addition to the self-locking of the limiting member 310 in cooperation with the rocker arm 420, additional locking structure can be provided for locking to prevent the moving member 410 from resetting which urges an undesired opening of the clamping arms 100.

In an embodiment, the clamping member 1 has a locking engaging portion, and the moving member 410 has a locking portion. When the clamping arms 100 are in the clamping state, the locking engaging portion and the locking portion relatively move to a locking position. When the clamping arms 100 are separated from the transmission member 510, the locking portion and the locking engaging portion can be locked in time to prevent the moving member 410 and the rocker arms 420 from moving towards the front end of the clamping member 1 and prevent the clamping arms 100 from opening.

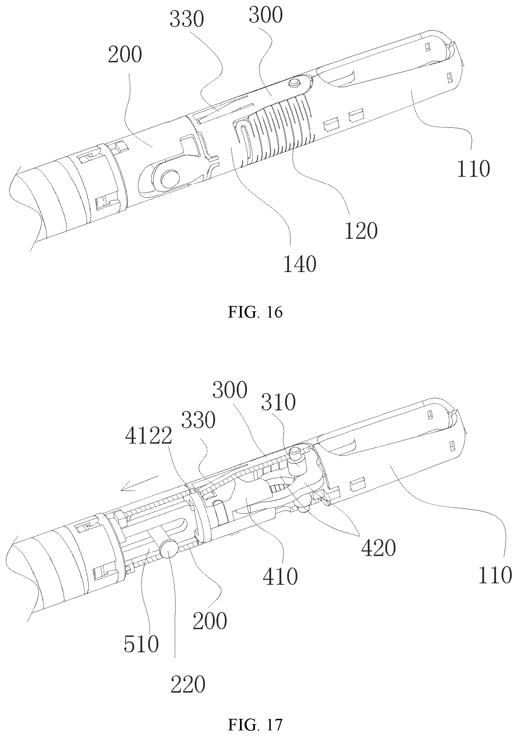

Referring to FIGs. 16 to 18, in an embodiment, the locking portion is an engaging platform 4122 provided on the moving member 410, and the locking engaging portion is an elastic buckle 330 extending inwardly. The elastic buckle 330 is provided in a path in which the engaging platform 4122 moves towards the back end. When the clamping arms 100 are in the clamping state, the engaging platform 4122 can move towards the back end to pass the position of the elastic buckle 330. When the clamping arms 100 are separated from the transmission member 510, under the elastic resetting force of the clamping arms 100, the engaging platform 4122 can be reset towards the front end with the moving member 410 by a small distance. When the elastic buckle 330 abuts against the front side of the engaging platform 4122, the engaging platform 4122 can be prevented from continuing to move towards the front end of the clamping member 1, thereby forming a locking.

Referring to FIGs. 16 to 18, in an embodiment, in order to better guide the movement of the elastic buckle 330 towards the engaging platform 4122, an outer wall of the engaging platform 4122 can form a slope surface 4122a extending along a longitudinal direction thereof.

Referring to FIGs. 16 to 18, in an embodiment, the elastic buckle 330 is formed by a partial region of the support arm 300 extending inwardly. The elastic buckle 330 is integrally formed with the support arm 300. This structure is simple to be manufactured, and can be integrally formed, for example, can be processed by laser cutting without adding other parts. Further, the elastic buckle 330 can utilize the space along the longitudinal direction, so that the elastic buckle 330 has a relatively long length and an improved elasticity when the clamping member 1 holds the same length.

In some embodiments, the two support arms 300 each can be provided with elastic buckles 330. When one support arm 300 is provided with the above-mentioned limiting guide portion, referring to FIG. 14, in an embodiment, one support arm 300 can have the elastic buckle 330, and the other support arm 300 can have the limiting guide portion (such as a guide groove 320) provided along the longitudinal direction thereof. The limit guide portion is configured to guide the movement of the moving member 410 along a longitudinal direction of the clamping member 1. In this embodiment, the locking condition of the elastic buckle 330 is unique, and the locking is more reliable. If two elastic buckles 330 are provided, it not only occupies space, but also allows the two elastic buckles 330 to be accurately aligned with lock the engaging platform 4122 and locked at the same time, which requires a relatively high precision in manufacturing and assembling parts. The limiting guide function of the limiting guide portion on the other side can make the elastic buckle 330 and the engaging platform 4122 of the moving member 410 radially swing in a relatively small range, thereby ensuring more stable locking.

Meanwhile, when the self-locking structure formed by the limiting member 310 and the rocker arm 420 is provided, the counter force applied by the target tissue 2 to spread the clamping arms 100 is converted into the transverse displacements of the rocker arms 420 relative to the limiting member 310 through the rocker arms 420, and the force applied to the moving member 410 towards the front end becomes very small, which also means that the elastic buckle 330 only needs to bear a small force towards the front end to realize the locking of the whole clamp. Therefore, usually one elastic buckle 330 can realize the locking.

Further, unlike the prior art which achieves opening and closing of the clamping arms 100 by limiting the clamping arms 100 via a sleeve, in this embodiment, opening and closing of the clamping arms 100 mainly depends on deformation of the bendable portion 120. The bendable portion 120 has a structure capable of bending in the closing direction of the clamping member 1 and/or bending in the opening direction of the clamping member 1. Referring to FIGs. 5 to 7, in the illustrated embodiment, the initial state of the clamping member 1 is the clamping state, that is, the clamping member 1 is in the clamping state without deformation of the bendable portion 120. In this case, the bendable portion 120 at least has a structure that can be bent in the opening direction of the clamping members 1, thereby realizing the opening of the clamping members 1 as shown in FIGs. 2 to 4.

The clamping head 110 is a rigid section and is less deformable than the bendable portion 120. During the movement of the moving member 410 towards the front end and the back end, the bendable portion 120 bends and deforms prior to the clamping head 110 to ensure that the clamping arm 100 provides a better occluding effect on the target object. The bendable deformation of the bendable portion 120 can be realized by structural deformation, for example, by providing a bending structure capable of twisting deformation on the bendable portion 120, by changing the material thickness of the bendable portion 120, or by selecting a material that is more easily deformed, or can be realized by other structures. The bendable deformation of the bendable portion 120 is reversible, that is, the bendable portion 120 has elasticity and can be rebound and returned when an external force is lost, so that such bendable deformation can be repeated.

Referring to FIGs. 2 to 7, in an embodiment, the bendable portion 120 has a semi-cylindrical structure. When the clamping arms 100 are closed, the bendable portions 120 can enclose into a cylindrical structure. The semi-cylindrical shape refers to an incomplete cylindrical shape, which does not have to be half of the cylindrical structure, but can be one-third of the entire cylindrical structure or other sizes. Further, in some other embodiments, the bendable portion 120 may be of other structures, such as a sheet, and is not limited to the semi-cylindrical structure.

Further, referring to FIGs. 1 to 7 and 19, in an embodiment, the bendable portion 120 includes a plurality of first shrinkage slit groups 121 and a plurality of second shrinkage slit groups 122. Each first shrinkage slit group 121 has at least one first shrinkage slit 1211. Each second shrinkage slit group 122 has at least one second shrinkage slit 1221. The first shrinkage slit 1211 and the second shrinkage slit 1221 extend in the circumferential direction of the bendable portion 120. The first shrinkage slit group 121 and the second shrinkage slit group 122 are spaced apart along the longitudinal direction of the clamping arm 100. One first shrinkage slit group 121 is provided between the two second shrinkage slit groups 122. The overlapping region between the first shrinkage slit 1211 and the second shrinkage slit 1221 forms the twisted deformation section 124 to enable the bendable portion 120 to be bent and torsionally deformed.

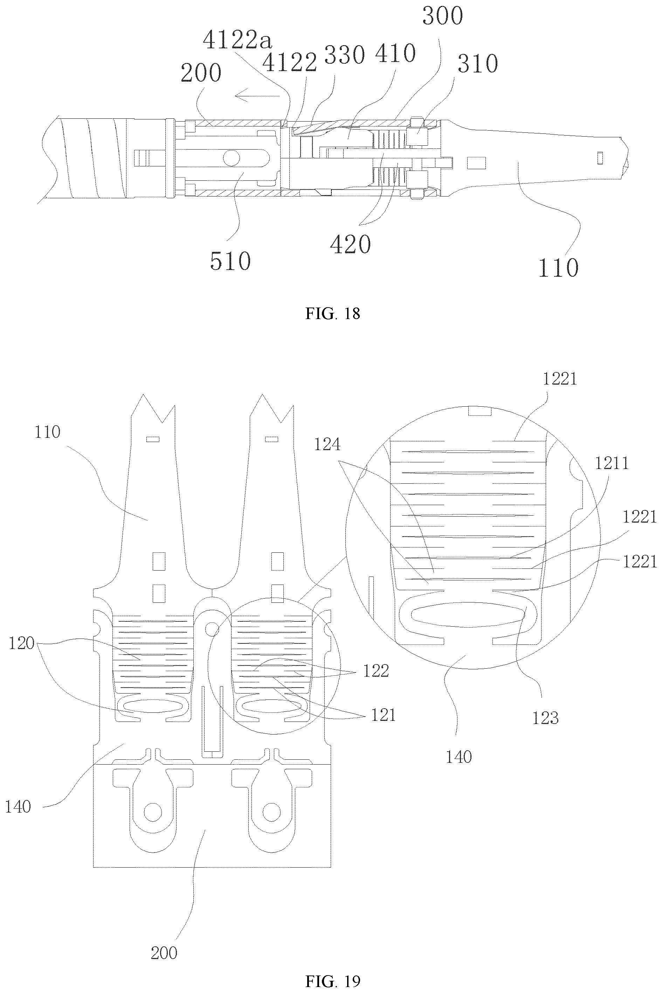

In an embodiment, as shown in FIGs. 5 and 19, the clamping member 1 is maintained in the clamping state in the initial state, the first shrinkage slits 1211 are maintained in the initial state, and each portion of the bendable portion 120 is not deformed. As shown in FIGs. 2 and 19, when it is necessary to open the clamping member 1, the bendable portion 120 deforms outwardly, the first shrinking slit 1211 and the second shrinking slit 1221 shrink, and the twisted deformation section 124 undergoes bending and twisting deformation, thereby shrinking the outer side of the bendable portion 120 (the side of the clamping arms 100 away from each other), so that the entire clamping head 110 is opened.

In an embodiment, the first shrinkage slit group 121 and the second shrinkage slit group 122 can be integrally formed by laser cutting on a tube or sheet. For example, laser cutting on a pipe or sheet having a wall thickness of 2 mm.

Referring to FIG.19, in an embodiment, two second shrinkage slits 1221 are provided between adjacent first shrinkage slit groups 121. The two second shrinkage slits 1221 are respectively provided on both sides of the bendable portion 120, and the second shrinkage slits 1221 extend outwardly to corresponding side edges of the bendable portion 120.

Along the longitudinal direction of the bendable portion 120, within the same distance, the twisted deformation sections 124 are distributed more and more densely, and accordingly the bendable deformation of the bendable portion 120 becomes increasingly flexible. Meanwhile, in order to take into account the strength of the bendable portion 120, in an embodiment, the number of the first shrinkage slit groups 121 is in a range from 6 to 10, and the second shrinkage slit groups 122 are distributed at both ends of the bendable portion 120, so that the number of the second shrinkage slit groups 122 is one more than that of the first shrinkage slit groups 121, and the number is in a range from 7 to 11. With this number, the bendable portion 120 can be easily bent, and the strength can be ensured, avoiding that the bendable portion 120 is too soft to support the clamping head 110. When the operator normally opens and closes the clamping member 1, only a small force is needed to realize the opening and closing with a good hand feel. As shown in FIG. 19, in the illustrated embodiment, the number of the second shrinkage slit groups 122 is 8, and the number of the first shrinkage slit groups 121 is 7. By adjusting the circumferential lengths of the first shrinkage slit 1211 and the second shrinkage slit 1221 and the distance between each other, the flexibility and the supportability of bending can be changed, and the circumferential lengths and the distance can be flexibly set as required.

Referring to FIG. 19, in an embodiment, the first shrink slits 1211 are provided in parallel with each other and the second shrink slits 1221 are provided in parallel with each other.

Certainly, the first shrinkage slit 1211 and the second shrinkage slit 1221 can be provided in a non-parallel arrangement in addition to being parallel to each other. By setting the first shrinkage slits 1211 to be provided in parallel in the circumferential direction of the bendable portion 120, the bending deformation direction of each of the first shrinkage slits 1211 can be consistent, so that the bending deformation of the clamping member 1 can be more smooth and stable.

Further, in an embodiment, the first shrinkage slit 1211 has a gap along the longitudinal direction of the bendable portion 120. Referring to FIG. 19, the first shrinkage slit 1211 is a long strip-shaped slot, and has two opposite convex arc-shaped edges at the middle portion of the first shrinkage slit 1211. When the clamping member 1 is opened to the limiting position, the arc-shaped edges can contact each other, thus determining the maximum angle of opening.

In the embodiment shown in FIG. 19, the second shrinkage slit 1221 is provided in a straight-line shape in the circumferential direction. In some other embodiments, the second shrinkage slit 1221 can also be provided in other shapes.

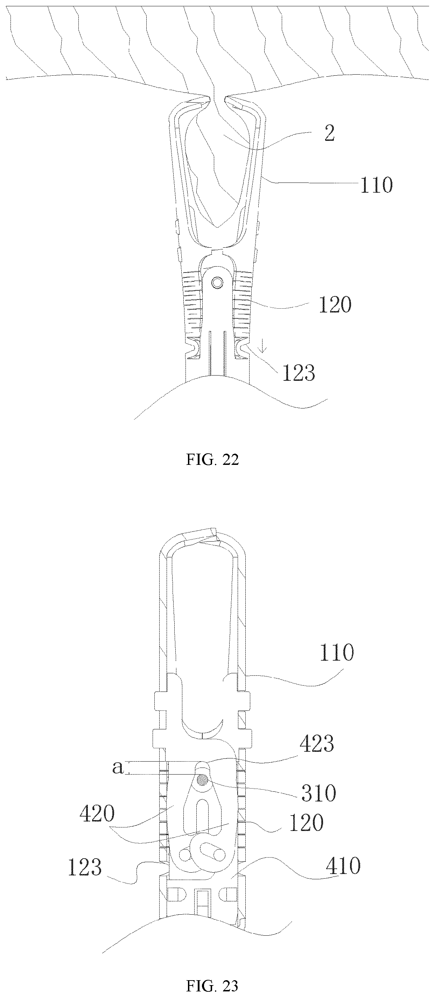

Further, referring to FIGs. 22 and 23, when the clamping arms 100 clamp the target tissue 2, the moving member 410 needs to move to a predetermined locking position together with the clamping arms 100 for locking, that is, the engaging platform 4122 needs to move to a position corresponding to the elastic buckle 330 for locking. However, in actual use, the hardness or thickness of the human tissue clamped by the clamping member 1 varies (as shown in FIG. 22), which affects the closing angle of the clamping member 1. Since that closing angle is associate with the stroke of the moving member 410, the moving member 410 cannot move to the locking position, and the clamping arm 100 cannot be maintained in the clamping state.

In view of this problem, referring to FIGs. 19 to 24, the bendable portion 120 has a ring-shaped deformation portion 123. When the clamping arms 100 are in the clamping state, if a larger target tissue 2 is clamped, it is difficult for the clamping arms 100, the moving member 410, and the rocker arms 420 to move to a position where self-locking with the limiting member 310 and locking with the locking engaging portion can be realized. When a relatively larger force is continuously applied to the transmission member 510, the deformation portion 123 can be extruded inwardly and deformed to drive the clamping arms 100, the moving member 410, and the rocker arms 420 as a whole to move towards the back end of the insertion-type tissue clamping device relative to the supporting portion and the limiting member 310, so that self-locking of the rocker arms 420 and the limiting member 310 and locking of the moving member 410 and the locking engaging portion are realized. A gap is provided between the deformation portion 123 and the support arms 300, so that an avoiding empty area is formed on the outer periphery of the deformation portion 123, thereby facilitating deformation.

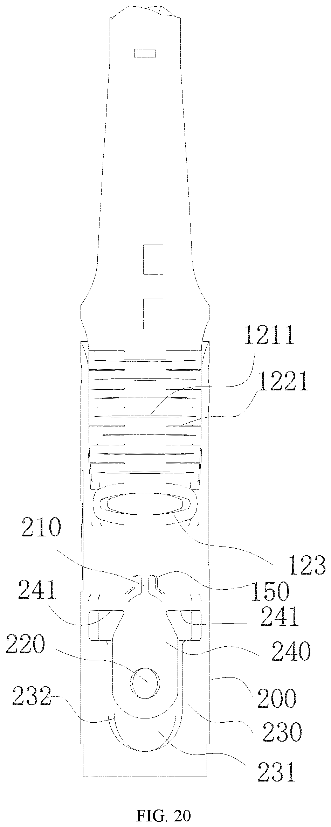

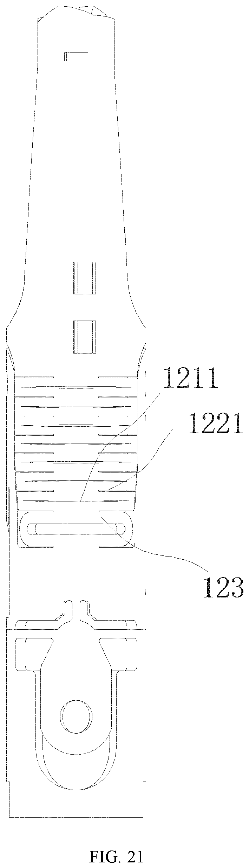

Specifically, referring to FIG. 20, when the clamping arms 100 clamp a thinner target tissue 2, the clamping arms 100 can be closed normally, the deformation portion 123 maintains a normal gap, and the moving member 410 and the clamping arms 100 can accurately move to the position of the locking structure as described above, and the clamping arms 100 can be locked in the clamping state. Referring to FIGs. 21 and 22, when the clamping arms 100 clamp a thicker target tissue 2, the clamping arms 100 cannot be closed to the extent shown in FIG. 20. In this case, the force is continuously applied to the moving member 410, and when the operator intentionally applies an enhanced force, the deformation portion 123 is greatly deformed, thereby compensating for the lost stroke on the clamping arms 100 and the moving member 410, so that the clamping arms 100 can finally be locked to the locking structure.

Referring to FIGs. 19 to 23, the deformation portion 123 provides an adaptive stroke range for the moving member 410 under different opening states of the clamping arm 100, generates a large deformation amplitude after receiving a specific pull-down force, always allows the elastic buckle 330 to be accurately and reliably locked, and allows the rocker arms 420 and the limiting member 310 to realize self-locking.

Specifically, the first shrinkage slit group 121 and the second shrinkage slit group 122 are provided on the front side of the deformation portion 123. The twisted deformation section 124 is connected to the front portion of the deformation portion 123, that is, the upper wall of the deformation portion 123 is connected to the twisted deformation section 124 of the clamping arm 100. The lower wall of the deformation portion 123 is connected to a connecting portion 140 (described in detail later), and the deformation portion 123 forms a gap with the support arm 300 in the transverse direction to avoid interference. When the deformation portion 123 is subjected to a force, the radian gradually increases, even in a straight-line state, and the quadrant points of the upper and lower walls may overlap with each other.

The deformation amplitude of the deformation portion 123 can be determined according to the difference between the actual stroke and the desired stroke between the rocker arm 420 and the limiting member 310 when the clamping arms 100 clamp the large target tissue 2, that is, as shown in FIG. 23, the distance between the rocker arms 420 and the limiting member 310 and the locking and self-locking position determines the deformation size of the deformation portion 123. After repeated experiments, in an embodiment, the deformation portion 123 has a maximum distance a of being extruded inwardly and deformed in a range of 0.1 mm≤a≤1.0 mm, for example, from 0.3 mm to 0.8 mm.

The deformable portion 123 is capable of adaptive floating deformation in the clamping direction, so that when the clamping member 1 clamps the target tissue 2, the bendable portion 120 can adaptively bend and deforms according to the volume of the target tissue 2, so as to provide an adaptive stroke range to the clamping arms 100, and allow the moving member 410 and the clamping arms 100 to move to the locking position at all times, thereby achieving accurate and reliable locking. The deformation portion 123 is designed such that the operation force does not cause the clamping member 1 to be locked when the operator normally opens and closes the clamping member 1, but the deformation portion 123 is greatly deformed only when the operator intends to release the clamping member 1, thereby stably and reliably locking the clamping member 1.

Referring to FIG. 19, in an embodiment, the deformation 123 is of an elliptical structure. Certainly, in some other embodiments, the deformation portion 123 can also be of other shapes, such as a circular structure or a diamond structure.

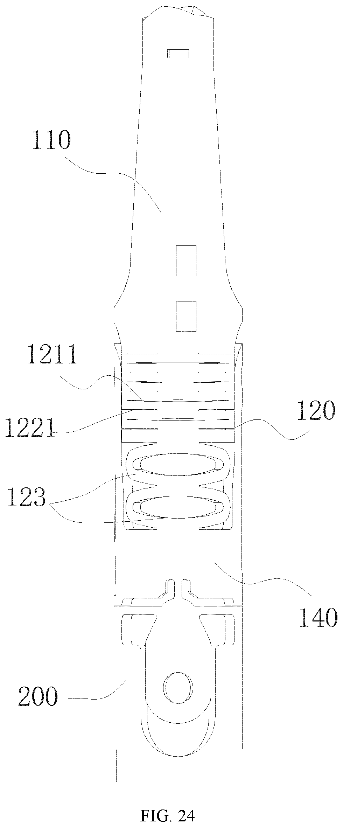

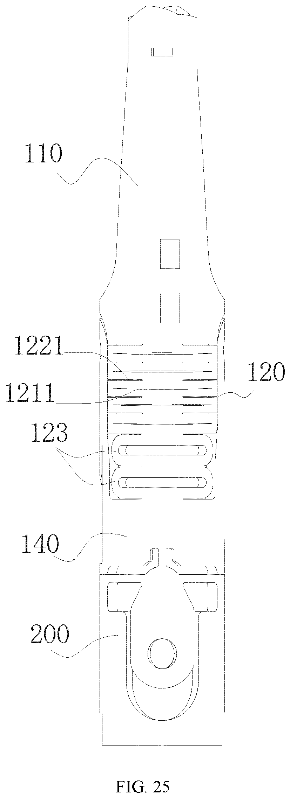

Further, the number of the deformation portions 123 is not limited to one. For example, referring to FIGs. 24 and 25, the number of the deformation portions 123 is also two or more, and these deformation portions 123 are sequentially connected along the longitudinal direction of the clamping arm 100. Through the superposition of the deformation of the plurality of deformation portions 123, each deformation portion 123 is slightly deformed when compressed inwardly, and finally a relatively large displacement towards the back end is obtained after superposition, which is used to compensate for the tolerance of the elastic buckle 330, so that it can be reliably locked, and the rocker arms 420 and the limiting member 310 can realize self-locking.

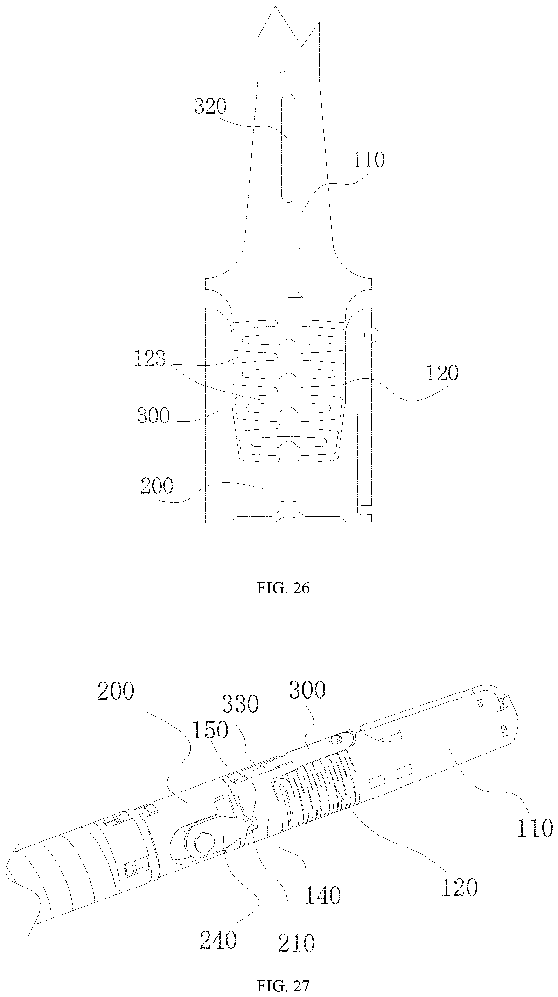

Further, referring to FIG. 26, in another embodiment, the first shrinkage slit group 121 and the second shrinkage slit group 122 described above can be omitted. The bendable portion 120 is composed of a plurality of deformation portions 123 that are sequentially connected along the longitudinal direction of the clamping arm 100. The opening and closing of the clamping arms 100 is achieved by the deformations of these deformation portions 123.

The above-described embodiment shows a structure in which the bendable portion 120 realizes bending deformation by providing the first shrinkage slit group 121, the second shrinkage slit group 122, and the twisted deformation section 124. The structure of the bendable portion 120 in the present embodiment is not limited to this, and can be realized by other means.

For example, in an embodiment, in the clamping arm 100, the thickness of the bendable portion 120 can also be set to be thinner than those of other portions, for example, thinner than those of the clamping head 110 and the connecting portion 140, so that the bendable portion 120 can preferentially bend and deform when the moving member 410 drives the clamping arm 100 to move.

In an embodiment, in the clamping arm 100, the bendable portion 120 can have a material and structure that are more easily bent and deformed, for example, a material that is more easily bent and deformed than those of the clamping head 110 and the connecting portion 140, such as a metal material, a plastic material, a wire woven mesh, and the like that have better bending performance. Thus, when the moving member 410 drives the clamping arm 100 to move, the bendable portion 120 can preferentially bend and deform. Certainly, the bendable portion 120 can also adopt other structures having better bending deformation performance, such as a metal braided structure.

Further, the first stroke, the second stroke, and the third stroke are three parts of the entire movement stroke of the transmission member 510. The three strokes can be in the same direction, or at least two strokes can be in different directions. The strokes can be completely separated, completely unrelated, or at least two strokes can be continuous or overlapped, for example, the third stroke can closely follow the second stroke. Certainly, the second stroke and the third stroke can also be two separate, non-continuous parts.

As an example, referring to FIGs. 2 to 4, the transmission member 510 is in the first stroke, the transmission member 510 moves away from the control handle 3 in the axial direction thereof and towards the front end, and drives the clamping arms 100 to open outwardly, thereby causing the clamping arm 100 to move to be in the opening state.

Referring to FIGs. 5 to 7, the transmission member 510 is in the second stroke, the transmission member 510 approaches the control handle 3 in the axial direction thereof and moves towards the back end, and the transmission member 510 can drive the clamping arms 100 inwardly towards each other, thereby causing the clamping arms 100 to move to be in the clamping state.

Referring to FIGs. 13 to 18 and FIGs. 27 to 32, the transmission member 510 is in the third stroke. When the transmission member 510 approaches the control handle 3 in the axial direction thereof and moves away from the clamping arms 100, the third stroke is in the same direction as and closely connected to the second stroke. That is, when the clamping arms 100 move to the clamping state, the transmission member 510 is switched from the second stroke to the third stroke. The third stroke can be divided into a plurality of sub-strokes including a locking stroke, an inner disengagement stroke, and an outer disengagement stroke.

Referring to FIGs. 16 to18, when the transmission member 510 is switched to the third stroke until it moves to the position shown in the figure, the clamping arms 100 are locked, and the transmission member 510 cannot move in the opposite direction and reopen the clamping arms 100. During this process, the movement stroke of the transmission member 510 is the locking stroke.

Referring to FIGs. 27 to 29, after completing the locking stroke, the transmission member 510 enters the inner disengagement stroke. When the transmission member 510 moves to the position shown in the figure, the clamping arms 100 are separated from the transmission member 510 at this time. The transmission member 510 can no longer drive the clamping arms 100 to move, and lose the control on the clamping arms 100, and the clamping arms 100 are maintained in the locking state. During this process, the movement stroke of the transmission member 510 is an internal disengagement stroke.

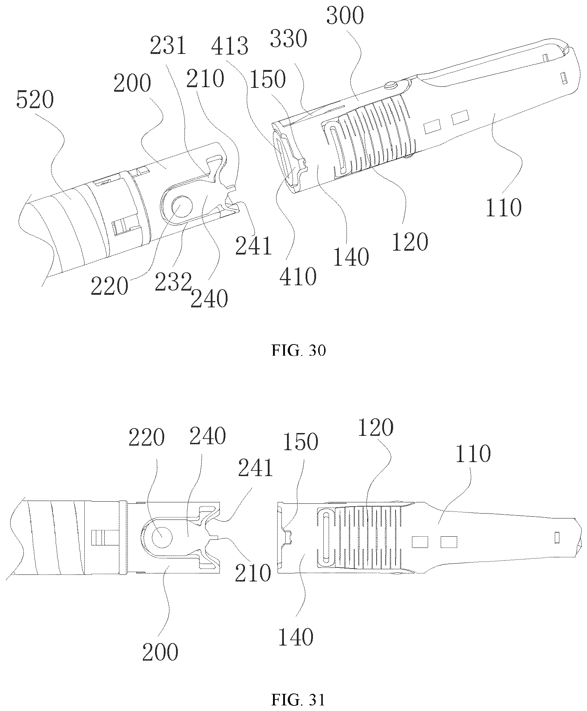

Referring to FIGs. 30 to 32, the transmission member 510 enters the outer disengagement stroke after completing the inner disengagement stroke. When the transmission member 510 moves to the position shown in the figure, the clamping arms 100 are disengaged from the separate base 200, so that the clamping arms 100 are left on the target tissue 2 clamped by the clamping arm 100. The separate base 200 and the transmission member 510 can be withdrawn from the patient. During this process, the movement stroke of the transmission member 510 is the outer disengagement stroke.

FIGs. 13 to 18 and FIGs. 27 to 32 only show one embodiment of the third stroke in which the separation of the clamping arms 100 from the transmission member 510, and the disengagement of the clamping arms 100 from the separate base 200 can be achieved simultaneously, or either action can be achieved before the other. In some other embodiments, the locking stroke, the inner disengagement stroke, and the outer disengagement stroke can also be performed concurrently, for example, the inner disengagement stroke and the outer disengagement stroke overlap, and the inner disengagement and the outer disengagement are performed synchronously.

To achieve inner disengagement, the present application provides an example in which the front end of the transmission member 510 has a first snap portion and the back end of the moving member 410 has a second snap portion. The first snap portion is snap-fitted with the second snap portion. At least one of the first snap portion and the second snap portion is of a deformable structure. When the transmission member 510 moves along the third stroke, the deformable structure can be deformed under the pulling force of the transmission member 510, so that the first snap portion is disengaged from the second snap portion, and the moving member 410 is separated from the transmission member 510.

The deformable structure refers to a structure that can be deformed only when the pulling force reaches a set value, and the structure can be realized by material and structural design, for example, the deformable structure is made of an elastic material.

One of the first snap portion and the second snap portion is a deformable connecting portion and the other is a fitting engaging groove, and the deformable connecting portion is snap-fitted in the fitting engaging groove. Referring to FIGs. 8, 28 and 29, the deformable connecting portion 511 is provided on the transmission member 510, and the fitting engaging groove 413 (which may be a through hole or a blind hole) is provided at the bottom of the moving member 410. The shape of the fitting engaging groove 413 is matched with the shape of the deformable connecting portion 511 to engage the deformable connecting portion 511.

Referring to FIGs. 8, 28 and 29, in an embodiment, the deformable connecting portion 511 includes at least one deformable hook that is connected in a corresponding fitting engaging groove 413. When the applied pulling force reaches the set value, the deformable hook can be pulled to deform and thus disengage from the fitting engaging groove 413.

The middle of the back end of the transmission member 510 can have a long slot, through which a limitation pin extends, and the bottom is cylindrical and connected to the pulling member (such as a pulling rope). When the pulling force of the pulling rope is greater than the bearing limit of the deformable hook, the deformable hook deforms inwardly, thereby disengaging from the moving member 410.

In order to achieve better engaging effect, referring to FIGs. 8, 28 and 29. In an embodiment, the deformable connecting portion 511 includes two deformable hooks, the two deformable hooks are arranged opposite to each other. The fitting engaging groove 413 can be provided with two cavities symmetrically communicating left and right. In addition, the deformable connecting portion 511 can also have at least one intermediate stopper 512. The intermediate stopper 512 is located between the two deformable hooks to prevent the two deformable hooks from excessively deforming to the opposite side to result in an uneven separation force.

Certainly, other inner disengagement structures can be used between the moving member 410 and the transmission member 510 instead of the structures shown in the above embodiments.

Further, referring to FIGs. 2 to 7, in an embodiment, the clamping arms 100 includes the connecting portion 140.The connecting portion 140 is provided on the back side of the bendable portion 120. The connecting portion 140, the bendable portion 120, the clamping head 110, and the support arm 300 are integrally formed. The connecting portion 140 is configured to connect the entire bendable portion 120, the clamping head 110, and the support arm 300 to the separate base 200. The separate base 200 and the connecting portion 140 can be integrally formed, or can be fixedly connected to each other after being manufactured separately.

In order to achieve outer disengagement, in an embodiment, referring to FIGs. 2, 30 to 32, the separate base 200 is rotatably connected to the sleeve assembly 520, so that the clamping member 1 can rotate relative to the sleeve assembly 520 as a whole. The clamping arms 100 and the support arms 300 are integrally connected to the separate base 200 by the first tearing portion 210. In this embodiment, the separate base 200 and the other parts of the clamping member 1 are integrally formed, and both are integrally connected by the first tearing portion 210. Additionally, the separate base 200 and other parts of the clamping member 1 can be connected as a whole by engagement or the like.

In an embodiment, referring to FIGs. 30 to 32, the separate base 200 has a following member 220. The following member 220 is configured to receive an external force to drive the separate base 200 to separate from other parts of the clamping member 1 at the position of the first tearing portion 210. For example, an external force applied by the operator can be transmitted to the first tearing portion 210 through a moving member 410 or other components.

Still referring to FIGs. 30 to 32, in an embodiment, the following member 220 is driven to move by a moving member 410. Specifically, the following member 220 is located on the moving trajectory of the moving member 410. When the moving member 410 moves to the position of the following member 220, it drives the following member 220 to move together towards the control handle 3, so that the separate base 200 is separated from the connecting portion 140 and the like at the position of the first tearing portion 210 by the action of the moving member 410.

Further, referring to FIGs. 30 to 32, in the outer disengagement structure, at least one first tearing portion 210 is provided, and the end of the entirety of the clamping arms 100 and the support arms 300 opposite to the separate base 200 has a concave region 150. For example, the concave region 150 is provided on the connecting portion 140. The first tearing portion 210 is provided in the concave region 150. The separate base 200 is connected to the clamping arms 100 and the support arms 300 only by the first tearing portion 210. This design enables the tearing surface of the first tearing portion 210 to be accommodated in the concave region 150 after the fracture of the first tearing portion 210, thereby avoiding exposure of sharp burrs after the fracture and avoiding damage to the surgical object.

Further, referring to FIGs. 20 and FIGs. 30 to 32, in an embodiment, the separate base 200 includes a cylindrical main body 230 and a suspension portion 240. The side wall of the body 230 has a suspension cavity 231. The suspension portion 240 is provided in the suspension cavity 231. The suspension portion 240 is aligned with and connected to the first tearing portion 210. The suspension portion 240 is provided with the above-described following member 220. For example, the following member 220 is a limiting shaft fixedly attached to the suspension portion 240, and the limiting shaft traverses the suspension portion 240. The following member 220 is configured to drive the suspension portion 240 and the transmission member 510 to move to the back end when the transmission member 510 moves along the third stroke. Both sides of the suspension portion 240 are connected to the main body 230 by suspension arm 241, so that the suspension portion 240 can be deformed with respect to the main body 230 to tear the first tearing portion 210.

Specifically, referring to FIGs. 30 to 32, the moving member 410 may also be provided with a limiting groove 513 disposed in the axial direction thereof. The following member 220 is disposed in the limiting groove 513. As the moving member 410 moves towards the control handle 3, when the moving member 410 enters the outer disengagement stroke, the top of the limiting groove 513 moves to the following member 220, thereby driving the following member 220 and the suspension portion 240 to move towards the control handle 3, thereby enabling the separate base 200 to separate from the connecting portion 140.

As a specific embodiment, referring to FIGs. 30 to 32, the following member 220 is a limiting shaft, and the limiting shaft is provided in the limiting groove 513.

In an embodiment, the width of the first tearing portion 210 can be reserved according to needs, and a plurality of first tearing portions 210 can be provided according to functional needs, so that the structure can be more reliable and stable.

In order to uniformly distribute the force, referring to FIGs. 30 to 32, in an embodiment, at least two first tearing portions 210 and at least two suspension portions 240 are provided. The first tearing portions 210 are distributed along the circumferential direction of the separate base 200. Each first tearing portion 210 is correspondingly provided with one suspension portion 240. Uniform distribution means that adjacent first tearing portions 210 or suspension portions 240 are spaced apart by the same distance or angle.

Furthermore, the following member 220 can also function as a stroke limit for the moving member 410 to limit the movement of the moving member 410 within a set range, so as to define the limit stroke of the moving member 410 towards the front end, thus defining the opening stroke of the clamping member 1. Referring to FIG. 3, when the limiting groove 513 moves forward to the position of the following member 220, the following member 220 blocks the transmission member 510, thereby defining the forward movement of the moving member 410 and the opening stroke of the clamping member 1.

Further, referring to FIGs. 30 to 32, in an embodiment, both sides of the suspension portion 240 are connected to the main body 230 by suspension arms 241, so that the suspension portion 240 can be more easily deformed relative to the main body 230.

Specifically, when the moving member 410 moves along the outer disengagement stroke, the entire separate base 200 cannot move to the control handle 3 alone under the support of the sleeve assembly 520. When the moving member 410 pulls the suspension portion 240, the main body 230 of the separate base 200 remains stationary, and the suspension arm 241 of the suspension portion 240 is deformed by the pulling force of the moving member 410. During the deformation of the suspension portion 240, the main body 230 of the separate base 200 forms a opposite support for the clamping arms 100 and the like, and the materials of the suspension arm 241 and the first tearing portion 210 are gradually elongated. As shown in FIGs. 30 to 32, when the yield limit is reached, fracture occurs, and the suspension portion 240 is disengaged from the connecting portion 140. Thereafter, the separate base 200 can be removed from the body of the surgical subject together with the transmission assembly 5.

In order to prevent the suspension portion 240 from deforming in an undesired direction when the moving member 410 pulls the suspension portion 240, referring to FIGs. 30 to 32, in an embodiment, the suspension cavity 231 has a guide groove 232 disposed in the axial direction of the separate base 200, and the suspension portion 240 is disposed in the guide groove 232 to guide the movement of the suspension portion 240 into the guide groove 232. The guide groove 232 defines a guide direction that is aligned with the first tearing portion 210, thereby allowing the suspension portion 240 to break from the first tear portion 210 more easily.

Those skilled in the art can recognize that many changes can be made to the details of the embodiments described above without departing from the basic principles of the present application. Accordingly, the scope of the present application should be defined by the appended claims.

## Claims

1. An insertion-type tissue clamping device, comprising:
a clamping member comprising at least two clamping arms and a separate base connected to the clamping arms, wherein the clamping arm comprises a clamping head and a bendable portion, the clamping head and the bendable portion remain relatively fixed to each other, and the clamping head is configured to clamp a target tissue;
a movement-controlling assembly comprising a moving member and at least two rocker arms, wherein the moving member is provided in the clamping member in such a way that the moving member is capable of moving in an axial direction of the clamping member, one end of the rocker arm is hinged on the moving member, and another end thereof is connected to one of the clamping arms, respectively;
a transmission assembly comprising a sleeve assembly and a transmission member, wherein the transmission member is connected to the moving member, and the separate base is connected to the sleeve assembly; and
a control handle connected to the sleeve assembly, wherein the control handle and the transmission member form a linkage structure to control movements of the transmission member and the moving member;
wherein the transmission member has a first stroke, a second stroke, and a third stroke; in the first stroke, the transmission member drives the clamping heads to be away from each other, such that the clamping arms move to be in an opening state; in the second stroke, the transmission member drives the clamping heads to approach each other, such that the clamping arms move to be in a clamping state to clamp the target tissue; and in the third stroke, the clamping arms remain in the clamping state, and the clamping arms are separated from the transmission member and the separate base.

2. The insertion-type tissue clamping device according to claim 1, wherein the clamping member comprises a support arm located between the clamping arms, the support arm is provided with a limiting member; when the moving member moves to a back end, the limiting member is located on a movement path of the rocker arms, and the limiting member is configured to contact at least one rocker arm and form a fulcrum of a lever structure for the at least one rocker arm.

3. The insertion-type tissue clamping device according to claim 2, wherein at least two rocker arms are arranged in an intersection-shape, and the limiting member is located in an intersection region formed by the two rocker arms and adjacent to the moving member, such that when the moving member moves to the back end, the limiting member forms the fulcrum of the lever structure for the at least one rocker arm.

4. The insertion-type tissue clamping device according to claim 3, wherein when the clamping arms move to be in the clamping state, a lever arm of a rotational center of the rocker arm relative to the moving member from a center of the limiting member is longer than a lever arm of a connection center between the rocker arm and the clamping head from the center of the limiting member.

5. The insertion-type tissue clamping device according to claim 3, wherein the two rocker arms have locking fitting surfaces, when the clamping arms are in the clamping state, the locking fitting surfaces of the two rocker arms enclose a locking groove, and the limiting member is located in the locking groove to prevent the two clamping arms from cross moving transversely, so as to maintain the clamping arms in the clamping state.

6. The insertion-type tissue clamping device according to claim 2 or 3, wherein a front end of the moving member has an angle limiting structure, when the clamping arms are in the opening state, the angle limiting structure forms a limitation on a back side of the limiting member to prevent the moving member from continuing to move towards a front end, so as to limit an opening angle of the clamping arms.

7. The insertion-type tissue clamping device according to claim 1, wherein a front end of the rocker arm away from a corresponding clamping arm has a concave portion, and the concave portion is configured to avoid a tissue clamped by the clamping heads.

8. The insertion-type tissue clamping device according to claim 2 or 3, wherein one of the moving member and the rocker arm has a rotating shaft, and another one has a self-adjusting groove, the rotating shaft is provided in the self-adjusting groove, and the rotating shaft and the self-adjusting groove are capable of adaptively adjusting relative positions of the moving member and the rocker arm in a transverse direction during movement of the rocker arm.

9. The insertion-type tissue clamping device according to any one of claims 2 to 8, wherein a front end of the rocker arm has a protrusion connecting portion, the protrusion connecting portion is inserted into a corresponding clamping arm and is fixedly connected to the clamping arm.

10. The insertion-type tissue clamping device according to any one of claims 2 to 9, wherein the moving member comprises a first seat body and a second seat body, the rocker arm is connected to and limited between the first seat body and the second seat body, the first seat body and the second seat body are spliced to form the moving member, and the moving member is slidably provided in the clamping member.

11. The insertion-type tissue clamping device according to any one of claims 2 to 10, wherein at least one support arm has a limiting guide portion provided along a longitudinal direction thereof, the moving member has at least one limiting guide engaging portion, and the limiting guide engaging portion is engaged with the limiting guide portion to prevent the moving member from moving along a direction defined by the limiting guide portion.

12. The insertion-type tissue clamping device according to claim 11, wherein one of the limit guide portion and the limit guide engaging portion is a guide groove, another one thereof is a guide block provided convexly, and the guide block extends into the guide groove.

13. The insertion-type tissue clamping device according to any one of claims 2 to 12, wherein a pair of the support arms are oppositely provided at a notch between two clamping arms, and both ends of the limiting member are fixedly connected to one of the support arms, respectively.

14. The insertion-type tissue clamping device according to any one of claims 1 to 13, wherein the bendable portion has a structure capable of bending towards a closing direction of the clamping arm and/or bending towards an opening direction of the clamping arm.

15. The insertion-type tissue clamping device according to claim 14, wherein the bendable portion has at least one deformable portion, when the clamping arms are in the clamping state, the deformable portion is capable of being extruded inwardly and deformed, so as to drive the clamping arms as a whole to move towards a back end of the insertion-type tissue clamping device.

16. The insertion-type tissue clamping device according to claim 15, wherein a gap is provided between the deformation portion and the support arm to form an avoiding empty area on an outer periphery of the deformation portion.

17. The insertion-type tissue clamping device according to claim 15, wherein the deformation portion has a maximum distance a of being extruded inwardly and deformed in a range of 0.1 mm≤ a≤1.0 mm.

18. The insertion-type tissue clamping device according to claim 15, wherein the deformation portion is in an elliptical shape.

19. The insertion-type tissue clamping device according to any one of claims 15 to18, wherein the bendable portion comprises a plurality of first shrinkage slit groups and a plurality of second shrinkage slit groups, each first shrinkage slit group has at least one first shrinkage slit, each second shrinkage slit group has at least one second shrinkage slit, the first shrinkage slit and the second shrinkage slit extend in a circumferential direction of the bendable portion, the first shrinkage slit group and the second shrinkage slit group are spaced apart along a longitudinal direction of the clamping arm, and an overlapping region between the first shrinkage slit and the second shrinkage slit forms a twisted deformation section to enable the bendable portion to be bent and torsionally deformed.

20. The insertion-type tissue clamping device according to any one of claims 2 to 19, wherein the clamping arm and the support arm are integrally formed or fixedly connected to each other.

21. The insertion-type tissue clamping device according to any one of claims 2 to 20, wherein the clamping member has a locking engaging portion, the moving member has a locking portion, when the clamping arms are separated from the transmission member, the locking portion and the locking engaging portion are locked to prevent the moving member and the rocker arms from moving towards a front end of the clamping member, so as to prevent the clamping arms from opening.

22. The insertion-type tissue clamping device according to claim 21, wherein the locking portion is an engaging platform provided on the moving member, the locking engaging portion is an elastic buckle extending inwardly, the elastic buckle is provided in a path in which the engaging platform moves towards the back end, when the clamping arms are separated from the transmission member, the elastic buckle abuts against a front side of the engaging platform to prevent the engaging platform from moving towards the front end of the clamping member.

23. The insertion-type tissue clamping device according to claim 22, wherein the elastic buckle is formed by a partial region of the support arm extending inwardly, and the elastic buckle is integrally formed with the support arm.

24. The insertion-type tissue clamping device according to claim 23, wherein one support arm has the elastic buckle, another support arm has the limiting guide portion provided along a longitudinal direction thereof, and the limit guide portion is configured to limit a longitudinal movement of the moving member along the clamping member.

25. The insertion-type tissue clamping device according to any one of claims 1 to 24, wherein a front end of the transmission member has a first snap portion, a back end of the moving member has a second snap portion, the first snap portion is snap-fitted with the second snap portion, at least one of the first snap portion and the second snap portion is of a deformable structure, when the transmission member moves along the third stroke, the deformable structure is capable of deforming under a pulling force of the transmission member, such that the first snap portion is disengaged from the second snap portion, and the moving member is separated from the transmission member.

26. The insertion-type tissue clamping device according to claim 25, wherein one of the first snap portion and the second snap portion is a deformable connecting portion, another one thereof is a fitting engaging groove, and the deformable connecting portion is snap-fitted in the fitting engaging groove.

27. The insertion-type tissue clamping device according to claim 25, wherein the deformable connecting portion comprises at least one deformable hook that is connected in a corresponding fitting engaging groove.

28. The insertion-type tissue clamping device according to any one of claims 2 to 27, wherein the clamping arm comprises a connecting portion provided on a back side of the bendable portion, and the connecting portion, the bendable portion, the clamping head, and the support arm are integrally formed.
